# EUROPEAN PATENT APPLICATION

(11) **EP 4 635 524 A1**
(43) Date of publication of application: **22.10.2025**
(21) Application number: 24170214.1
(22) Date of filing: 15.04.2024
(51) Int. Cl.: A61L 27/20, A61L 27/52, A61L 27/54

(54) **HYALURONIC ACID BASED HYDROGELS, DERMAL FILLER COMPOSITIONS AND PROCESSES FOR MAKING SAME**

(71) Applicant: Silimed Industria de Implantes Ltda., 21240-660 Rio de Janeiro / RJ (BR); Nanobioplus Pesquisas Tecnologicas Ltda., 13098-325 Campinas SP (BR)
(72) Inventor: ANDRADE SANTANA, Maria Helena, 13085-055 Campinas-SP (BR); GOMES DE MELO, Bruna Alice, 13098-426 Campinas-SP (BR); ANDRADE SANTANA, Guilherme, 13085-055 Campinas-SP (BR)
(74) Representative: Vossius & Partner Patentanwälte Rechtsanwälte mbB

(57) **Abstract**

The invention relates to a process for fabricating Hyaluronic Acid (HA) injectable dermal fillers. The process consists of crosslinking HA of high molecular weight (MW), particularly 10⁶ Da, with the crosslinking agent 1,4-butanediol diglycidyl ether (BDDE) with a lower amount of BDDE through an intensified reaction in a planetary centrifuge under vacuum or with higher BDDE amount in a conventional and less efficient process that uses mechanical stirring for the reaction and an ultra-turrax equipment for particle sizing. The processes generate HA hydrogels with only one degree of crosslinking that can be used to fabricate a wide range of HA dermal fillers with varied HA concentrations and particle size combinations. HA dermal fillers manufactured herein are based on a new technology called particle balance technology (PBT), in which hydrogels with an extensive range of physicochemical and rheological properties can be obtained through the combination of large and small particle sizes. Besides being used in medical surgery and orthopedic applications, these hydrogels can be used for dermal filling, body and face contouring, skin volumization, and hydration.

## Description

### Field of the Invention

The present invention pertains to dermal filler compositions and, more specifically, to injectable dermal fillers including Hyaluronic Acid (HA) hydrogels and their process of preparation.

### Background of the invention

Hyaluronic acid (HA) is a naturally occurring compound found in various parts of the human body, including the skin, joints, and eyes. HA belongs to the glycosaminoglycan family and serves as a fundamental component of the extracellular matrix, imparting crucial structural support to cellular dynamics and tissue functionality.

HA possesses unique physical properties that make it a versatile and pivotal compound for the skin. HA exhibits a great capability to retain water molecules, playing a fundamental role in tissue hydration, thereby contributing to the skin elasticity, firmness and overall appearance. Its viscoelastic nature plays a crucial role in tissue lubrication and safeguarding of cellular structures, promoting tissue health and vitality.

HA signaling prompts the production of collagen, an essential protein for tissue strength and integrity. This collagen-inducing effect is of prime importance in rejuvenating and revitalizing the skin. HA also signals for its endogenous own production for self-renewal, thus restoring its equilibrium between degradation and synthesis.

These properties have boosted HA into extensive use in aesthetic procedures to reduce the appearance of wrinkles and add volume to various areas of the face. Additionally, HA has a central role as a common ingredient in a wide array of skincare products, encompassing moisturizers, serums, and topical beauty formulations.

The multifaceted potential of HA in hydration, lubrication, and cell signaling, justifies its relevance across diverse applications, prominently including the subject matter of the present invention.

The process of preparing HA fillers involves several steps (Rzany B., 2018; Narins R.S., 2016; Burgess C.M. 2018; Micheels P. 2019).

HA can be obtained from various sources, including fermentation using native or genetically modified bacteria or extraction from rooster combs. The most common method used by manufacturers is bacterial fermentation. The HA is typically a sodium salt (NaHA) that is thermodynamically more stable than its acidic form.

Once obtained, the HA goes through serial purification processes to remove impurities, such as proteins, nucleic acids, and endotoxins. Purification methods may include filtration, adsorption, and precipitation techniques. The aim is to obtain a highly pure form of HA (>99.5%purity).

HA fillers often undergo a reaction called crosslinking, involving the formation of chemical bonds between HA chains mediated by a crosslinker agent. The crosslinking agents used can vary, but commonly used agents include 1,4-butanediol diglycidyl ether (BDDE) and divinyl sulfone (DVS).

BDDE is the most widely used agent of dermal fillers. It forms covalent bonds with the hydroxyl groups on the HA disaccharides, creating a three-dimensional network of interconnected HA chains.

Crosslinking is the crucial preparation stage because it forms the hydrogels that provide the physicochemical and rheological properties of the dermal fillers. It also provides resistance to enzymatic degradation and oxidation by free radicals, promoting long-lasting effects at the application site.

The crosslinking reaction between HA and the crosslinking agent typically occurs under controlled conditions. Factors such as temperature, pH, reaction time, and stirring speed are carefully optimized to ensure the desired level of crosslinking while maintaining the integrity and biocompatibility of the HA filler.

The degree of crosslinking drives the desired properties of the final HA filler. It is controlled by the molar proportion (which may also be referred to as molar ratio) between the crosslinking agent and the HA monomer, and the reaction conditions. The yield of the process defines the modification degree of HA, also referred as crosslinking degree (or degree of crosslinking), which is the percentage of the polymer chain modified by the crosslinking agent. This modification provides hydrogels with distinct physicochemical and rheological properties. Higher crosslinking degrees generally result in a more robust, stiffer, and longer-lasting filler, while lower levels of crosslinking may provide a softer and more easily moldable product.

The amount of BDDE in the reaction must be sufficient to ensure an effective crosslinking reaction. BDDE-HA molar ratio (1 BDDE chain to 1 HA disaccharide) usually varies from 0.1:1 to 2:1, depending on the desired properties of HA filler, softer to stiffer, respectively.

The most common technique for the crosslinking reaction between HA and BDDE is mechanical mixing using an impeller, which can be followed by an oven step. This process can take several hours. However, the crosslinking of HA with BDDE can be intensified by higher mixture efficiency using a planetary centrifuge, reducing the processing time.

For example Galderma for Restylane^{®} and Allergan for Juvederm^{®} use their proprietary technologies. Both products preferably use HA in its salt form, for example the sodium hyaluronate (NaHA). Then the saline form is crosslinked in an alkaline medium with a BDDE, which interconnects the HA chains through a chemical reaction with its hydroxyl group (US11058640B1, US20160376382A1, US8394782B2, Kenne L. 2012; Faivre J. 2021; Xue Y. 2020).

Galderma owns NASHA (Non-Animal Stabilized Hyaluronic Acid) technologies, which uses high molecular weight (MW) viscous HA, physically crosslinked only by intertwining its chains or with very low chemical crosslinking to obtain firmer textures and a more pronounced lifting (Micheels P., 2016; Edsman K., 2012; Kablik J., 2016).

Additionally, Galderma also uses OBT (Optimal Balance Technology) technology, which combines the balance between HAs with physical crosslinking and the viscoelastic HA with chemical crosslinking with BDDE to obtain softer textures, which allow for smoother integration with the tissue, providing a more natural look (Micheels P., 2016).

Allergan uses Hylacross and Vycross technologies in its product line. Hylacross technology uses high ratio of high MW HA to lower MW HA, with different crosslinking degrees and BDDE-HA molar ratio (0.1: 1 to 0.5:1), resulting in smooth and malleable gels with a homologous consistency (Brandt, 2008; Duranti, 1998; US6921819B2).

Vycross technology uses a higher proportion of low MW HA to higher MW HA, resulting in an efficient crosslinking. The product has low swelling capacity, enhancing smoothness and longer duration (Segurado, 2021; Brandt 2008; Fallacara, 2017).

The Galderma brand uses various crosslinking degrees in HAs with low and high MW, obtaining different particle sizes using sieving (low shear) (Fundarò, 2022).

The Allergan brand uses one single crosslinking degree in both low and high MW HAs, with low MW HA being in greater proportion, generating firmer particles by crosslinking the softer, low molecular weight HA with the crosslinking of high molecular weight HA (US7741476B2).

Following the crosslinking reaction, the resulting HA hydrogel undergoes a critical series of processes fundamental to determining its composition and properties. First, the hydrogel is broken (micronized) by low shear mixing and washed with a phosphate buffered saline (PBS) to remove excess of BDDE and swelled (water absorption) until it attains the hydration balance which defines its ultimate mass. This final mass defines the HA concentration within the hydrogel and plays a pivotal role in shaping the hydrogel properties.

Subsequently, the swelled HA hydrogel can be submitted to a workup using instrumental techniques to obtain crosslinked particles with different microscopic sizes, creating products in the same line that differ in texture, cohesiveness, viscoelasticity and flexibility, and thereby meeting specific applications and/or functions.

The most frequently employed techniques are sieving, jet milling, and high shear dispersion using ultra-turrax equipment.

Sieving entails passing the washed and swelled HA hydrogel through meshes with specific sizes, yielding particles of varying diameters while isolating particles within a desired size range (in other words, desired diameter range). This process is indispensable for achieving consistent particle size distribution (which is preferably to be understood as information on how many particles are characterized by different average size or average diameter), a factor that profoundly influences the properties and performance of the resulting dermal filler.

Jet milling, on the other hand, utilizes high-speed compressed gas or air jets to break down larger particles into smaller, more uniform ones. This milling technique can achieve targeted particle size distributions in HA hydrogel preparations.

High shear dispersion with ultra-turrax equipment operates on the principle of a rotor-stator system. Here, the high axial velocity draws the hydrogel towards the head of the dispersing element, forcefully propelling it with the assistance of the stator teeth. This dynamic process results in a hydrogel dispersion, with the shear force exerted at high speeds being adjustable according to the duration and intensity of its application. This capability allows for the control of particle sizes, i.e. the particle size distribution to be obtained in the process.

In this context, a high crosslinking degree of the hydrogel may produce a line of products with firmer textures, varying the particle diameter that make up its formulations. Alternatively, a low crosslinking degree may produce a range of products with softer textures by changing the particle diameter that make up its formulations. Differences in their properties of cohesiveness, viscosity, viscoelasticity, durability, and flexibility go along with the texture of product lines (Choi, 2020).

For Galderma, there is also the combination of many crosslinking degrees with many particle sizes (calibration) obtained by sieving, which distinguish products for deeper and more superficial fillings. Thus, bulkier (thicker) or thinner hydrogels are obtained by varying the particle size calibration and firmer or softer hydrogels by varying the crosslinking degree.

To further enhance dermal filler extrusion and safeguard HA hydrogel particles from degradation, non-crosslinked HA can be introduced into the HA hydrogel formulation.

Galderma, Allergan and also other brands use high and low MW HAs and combine chemically crosslinked and non-crosslinked HAs. As apparent to the skilled person, such HA-comprising fillers may be provided in a form of prefilled syringes. The total mass of hyaluronic acid in the syringe is adjusted with non-crosslinked HA. As regards the commercial products with high and low MW and one crosslink, low MW crosslink more than high MW, the change of rheological properties is monitored with a concentration of crosslinked and non-crosslinked HA. Typically, 1% w/w of non-crosslinked HA is added.

Other commercial products use various grades of crosslinking for one MW and adjust rheological properties with HA concentration; others use methods comprising various steps of crosslinking in sequence. These strategies change the rheological property viscoelasticity, driving the products for the various functions, such as volumizing, superficial wrinkles, etc.

Hydrogels from such brands contain an anesthetic. The anesthetic is typically introduced at the end of the process. For example, lidocaine in an acidic medium (lidocaine HCl), is added and vigorously homogenized in the hydrogels, increasing its cohesiveness and providing well-being to the patient by alleviating pain during the application.

The final step in the manufacturing process involves sterilizing the HA hydrogel to ensure its safety. Sterilization methods typically include gamma irradiation or autoclaving, which effectively eliminate potential contaminants.

The sterile filler is then packaged in pre-filled syringes or vials, ready for clinical use. This comprehensive process ensures that the HA hydrogel meets the highest quality and safety standards, making it suitable for medical and aesthetic applications.

The current preparation of HA for dermal fillers is a time-consuming process. From a HA previously fabricated by fermentation or extraction, highly purified and with its average molecular weight and distribution characterized, the preparation of dermal fillers involves multiple steps, such as crosslinking, washing, particle sizing, adjustment of the final mass with non-crosslinked HA, sterilization and packing in syringes.

Despite these advances in the field, there remains a need for alternatives for the processes and optimizations of the steps for preparation of dermal fillers.

### Summary of the Invention

The present invention relates to advancements in the manufacture of HA hydrogels, focusing on their compositions and fabrication processes (i.e., production/preparation processes) for injectable dermal fillers using the innovative "Particle Balance Technology" (PBT).

The PBT uses HA (preferably in the form of a salt, in particular its sodium salt, i.e. NaHA; it is to be understood that, preferably, the MW values refer to the MW of said NaHA) with only one HA MW (e.g., having an average MW in the range from 10⁵ Da to 10⁶ Da, for example MW being on average 10⁶ Da) The PBT uses HA having an average MW of the magnitude order 10⁶ Da, which is composed of HA fractions with MWs in the range of 10⁵ to 10⁷ Da, preferably in the range of 10⁵ to 10⁶ Da and one degree of crosslinking (in only one crosslinking step) with different proportions of particle sizes (which may be understood e.g. as the ratio of number of particles of one particular defined size to the number of particles of another particular defined particle size). It is particularly preferred that HA is in its salt form, in particular its sodium salt, i.e. NaHA, for the crosslinking reaction. It is to be understood herein that reference to MW or molecular weight (which may also be referred to as an average MW) preferably refers to weight average molecular weight. As it is apparent to the skilled person, weight average molecular weight may be determined for example by using mass spectrometry (MS) or size-exclusion chromatography (SEC) against a standard. Particularly preferred are measurements using SEC, which determines the average MW of HA and percentage of its fractions. The balance of particle sizes (i.e. presence of particles of different average sizes - or different average diameters - and particular ratio of their numbers) controls essential features of the hydrogels, such as texture, hydration degree, cohesiveness, and viscoelasticity, allowing them to offer an extended range of rheological and physicochemical properties to the dermal fillers.

According to the invention, the properties of the hydrogels are driven by the manufacturing process which comprises particular method steps of crosslinking reaction, which depends mainly on the ratio HA - BDDE and the efficiency of the reaction, obtaining of the particles, and the production of the PBT hydrogels.

In a first manufacturing process, the present invention uses a planetary centrifuge mixing equipment under vacuum for intensification of the crosslink reaction, allowing an extensive range of rheological and physicochemical properties with a lower amount of the crosslinker BDDE, such as 0.075:1 (crosslinker to HA molar ratio), and shorter times of reaction due to high mixture efficiency, in comparison to those of the commercial dermal fillers.

The low BDDE amount prevents adverse effects caused by the crosslinker, besides reducing or eliminating the residual non-crosslinked HA, as well as avoiding exhaustive washing after crosslinking, which results in better quality products.

The particles are obtained by sieving using different meshes to get a balance of different proportions of large and small particles of desired sizes. The flexible particles have irregular shape with low polydispersity relative to spherical shape, being characterized by an average diameter, as shown in Table 1.2, and a soft surface.

The final product also includes the addition of non-crosslinked HA to improve cohesiveness and extrusion of the gel. Typically, about 1% w/w of non-crosslinked HA is added.

These innovations in the fabrication processes make the manufacture of dermal fillers more efficient and less time-consuming.

The products of the present invention are cohesive and allow the use of a wide range of needles (27-32G), with extrusion force suitable for dermal filler application, as shown in Table 3.

In a second manufacturing process, the crosslinking reaction is carried out with the usual method of mechanical mixing, such as using a turbine impeller in a tank at a controlled temperature. The BDDE-HA molar ratios are in the range of commercial dermal fillers, 0.1: 1 to 0.5:1. The particles are obtained by high shear dispersion using ultra-turrax equipment at different speeds and times of applications. The particles are of the "crunch" type, with lower sphericity compared to the sieving method, and a rough surface due to the high shear forces applied. The final product also includes the addition of non-crosslinked HA to get the total mass of HA into the syringe with the desired physicochemical and rheological properties.

The products from both manufacturing processes may incorporate an anesthetic drug such as lidocaine to minimize pain during application besides the effects of its gradual delivery from the hydrogels.

The present invention is further described by the following embodiments.

In a first embodiment, the present invention relates to a process of fabrication of a crosslinked hyaluronic acid (HA) hydrogel comprising a mixture of two particle sizes of 150 µm and from 400 µm to 800 µm and having an HA concentration of 12 to 24 mg/ml, the process comprising the following steps:
a/ crosslinking of HA characterized by average MW of 10⁶ Da with molar ratio of crosslinking agent 1,4-butanediol diglycidyl ether (BDDE) to HA of 0.075: 1;
b/ conducting reaction of HA and BDDE in a planetary centrifuge under vacuum for less than 1 hour;
c/ washing the crosslinked HA and swelling to achieve the HA concentration of from 12 to 24 mg of HA per g of gel;
d/ homogenizing the crosslinked and swelled HA into particles using sieves of different mesh sizes and obtaining gel particles of two specific diameters between 150 and 800 µm, as measured by optical microscopy;
e/ isolating a batch of HA particles with an average diameter of 150 µm and a batch of HA particles with an average diameter ranging from 400 to 800 µm;
f/ combining particles characterized by a diameter of 150 µm with particles of diameter from 400 to 800 µm to fabricate the hydrogels; and
g/ mixing particles of different sizes (i.e. different average diameters) in a planetary centrifuge.

In a second embodiment, the present invention provides a process of fabrication of a crosslinked hyaluronic acid (HA) hydrogel, which comprises the steps of:
h/ crosslinking of HA (average MW 10⁶ Da) with the crosslinking agent 1,4-butanediol diglycidyl ether (BDDE) in the range of 0.1:1 to 0.5:1 BDDE-HA molar ratios containing only one crosslinking degree and preparation of hydrogels.

In a third embodiment, the present invention relates to a cross-linked HA hydrogel with a balance of particles size obtainable according to the any one of the processes described above.

In a fourth embodiment, the present invention relates to an aqueous composition comprising the cross-linked HA hydrogel of the present invention, and optionally a buffering agent and/or a tonicity agent.

In a fifth embodiment, the present invention relates to a pre-filled syringe, which is pre-filled with the cross-linked HA hydrogel of the present invention or the aqueous composition of the present invention.

In a sixth embodiment, the present invention relates to the crosslinked HA hydrogel of the present invention for use in cosmetic surgery (e.g., in dermal filling, body contouring, or facial contouring), in medical surgery (e.g., dermal filling, body contouring, prevention of tissue adhesion, formation of channels, incontinence treatment, or orthopedic applications), or for hydrating and/or vitalizing the skin.

In a seventh embodiment, the present invention relates to a cosmetic (non-therapeutic) use of the crosslinked HA hydrogel of the present invention as a dermal filler for the treatment (or reduction) of wrinkles.

In an eighth embodiment, the present invention relates to a dermal filler containing the crosslinked HA hydrogel of the present invention.

### Description of the Figures

Figure 1 shows (A) images of different particle diameters obtained after HA gel homogenization in different sieves and (B) PBT hydrogels resulting from the combination of small and large particles with different sizes at a ratio of 1:4. Scale = 500 µm.
Figure 2 shows scheme of general PBT process.
Figure 3 shows ¹H NMR spectra of degraded PBT hydrogel showing its components

### Detailed Description of the Invention

Although methods and materials similar or equivalent to those described herein can be used in the practice or testing of the present invention, suitable methods and materials are described below. All publications, patent applications, patents, and other references mentioned herein are incorporated by reference. The publications and applications discussed herein are provided solely for their disclosure before the filing date of the present application. Nothing herein is to be constructed as an admission that the present invention is not entitled to antedate such publication by the prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in the art to which the subject matter belongs. The following definitions are supplied herein to facilitate understanding of the present invention.

The manufacturing processes, according to the invention based on the Particle Balance Technology (PBT), allow to obtain HA particles with different diameters, from 150 to 800 µm, and then combine particles of HA gel of two different sizes to get a hydrogel with required the physicochemical and rheological properties.

In the first manufacturing process (or in other words first stage or first step of the process of the invention) for obtaining said hydrogels uses initially low BDDE-HA molar ratio, preferably in the range from 0.05: 1.0 to 0.1:1.0, more preferably in the range from 0.07: 1 .0 to 0.08: 1.0, such as 0.075:1.0, and crosslinking reaction is performed, for example in a planetary centrifuge under vacuum (i.e., under the pressure of less than 1 atm, most preferred 0.1 atm), which intensifies the reaction and optimizes the time, allowing a rapid process (for example, 45 min). Hydrogel is washed to remove residual BDDE (so that the residual content is up to 2 ppm) for 96 h and swelled until the desired HA concentration is reached. Then, hydrogel is homogenized in sieves of different mesh sizes (for a non-limiting example, as exemplified herein, the swelled hydrogel is homogenized into large particles using 60, 40, or 20 mesh sieves to obtain particles with 400, 600, or 800 µm of diameter, respectively), and particles are mixed in a planetary centrifuge for 10 min. Non-crosslinked HA (1% w/w) is added to the mixture and incorporated into the particles using a planetary centrifuge for 10 min.

Particles can also be obtained, alternatively to the use of planetary centrifuge, as described in the foregoing, by using ultra-turrax equipment, also known as a high-speed homogenizer or a rotor-stator homogenizer. This is a device commonly used in laboratory settings for mixing, emulsifying, dispersing, and homogenizing various substances, including hyaluronic acid.

Such products may also optionally incorporate (i.e., comprise) the anesthetic, such as lidocaine, for minimization of pain during application. Incorporating anesthetic lidocaine in hydrogels composed of different particle sizes may generate different profiles of controlled release (immediate release and modified release, e.g., lasting over certain time), providing greater comfort to the patient in its application.

The manufacturing processes according to the invention allows obtaining a plurality of combinations of particles and concentration of HA in the hydrogels. The balance of particle sizes (i.e., which is defined by particle size distribution) controls essential features of the hydrogel such as the products' texture, cohesiveness, viscoelasticity, and flexibility, making them stand out in terms of specific functions of dermal fillers application sites.

Measures of diameters and polydispersity are made using optical microscopy. Both diameter and polydispersity are average values because the particles are not rigid, but flexible particles. Accordingly, as it is to be understood herein, a reference to particle size (or average particle size) may be understood as a reference to its average, as shown in Table 1.2. As it is apparent to the skilled person, this parameter can be measured, for example, by using optical microscopy, or in dynamic light scattering (DLS) experiment. Preferably, this parameter (or average particle size/average particle diameter) is measured by optical microscopy.

In one embodiment of the process according to the invention, the preparation of a hydrogel comprising a mixture of two particles (i.e., two different types of particles) of HA with diameters from 150 and 800 µm and HA concentration of 12 to 24 mg/g, comprises the following steps:
a/ crosslinking of HA characterized by MW (to be understood as weight average molecular weight) in the range from 10⁵ to 10⁶ Da (preferably 10⁶ Da) with the crosslinking agent BDDE, achieved using a BDDE / HA molar ratio of between 0.05:1 to 0.10:1, preferably of 0.075 :1, preferably wherein b/ the reaction of HA and BDDE is conducted in a planetary centrifuge under vacuum for less than 1 hour;
c/ washing the crosslinked HA and swelling to achieve HA concentration of from 12 to 24 mg/g (i.e. mg per g of gel);
d/ homogenizing the crosslinked and swelled HA gel in sieves of different mesh sizes to obtain particles between 150 to 800 µm in diameter, as measured by optical microscopy;
e/ isolating HA particles with different diameters to obtain a batch of HA particles of higher diameter, i.e., large particles, and a batch of HA particles of lower diameter, i.e. small particles, wherein the average diameter of large particles is higher than average diameter of small particles; (it is to be understood that a batch of HA particles with an average diameter of 150 µm and a batch of HA particles with an average diameter ranging from 400 to 800 is obtained)
f/ combining particles of HA of different sizes (i.e., different average diameters) in the range from 150 and 800 µm to HA hydrogels; and
g/ mixing the particles of different sizes from both batches obtained in e, preferably in a ratio of 1:4, understood as number ratio of small to large particles in a planetary centrifuge.;
Optionally, the process may further comprise the step of mixing non-crosslinked HA (1% w/w) in a planetary centrifuge into the composition of g/.

According to the invention, the process allows for the quick and reproducible obtaining of a wide range of hydrogels and fine step control to get a mixture of HA with expected physicochemical and rheological requirements.

The preferred source of HA is the bacterial fermentation. HA is classified as high MW, with an average MW of 10⁶ Da (range of 10⁵ to 10⁷ Da).

In an alternative embodiment, the process of the present invention comprises the steps of: Crosslinking of HA (average MW 10⁶ Da) with the crosslinking agent 1,4-butanediol diglycidyl ether (BDDE) in the range of 0.1:1 to 0.5:1 BDDE-HA molar ratios and preparation of hydrogels using only 1 crosslinking degree.

The reaction of HA and BDDE is preferably to be conducted in a reactor with mechanical stirring at controlled temperature and a processing time higher than 1 hour. This process is followed by obtaining particles using a high-shear homogenizer, such as ultra-turrax equipment, with pre-calibrated rotation speed and application time.

Optionally, the process may further comprise the step of mixing non-crosslinked HA (1% w/w) in a planetary centrifuge into the obtained composition.

The present invention, in one embodiment, relates to a crosslinked HA hydrogel obtainable by any one of the methods of the present invention, as provided herein.

According to the present invention, The hydrogels may be sterilized inside the syringes in an autoclave, e.g., at 121 °C for 5 min. It was found that a product thus treated preserved its rheological properties and was not susceptible to bacteria and fungi growth.

Cellular characterizations should differ, such as signaling through particles for endogenous HA and collagen production. The efficiency of signaling depends on the particle size and surface properties.

In the subject matter of the invention, the PBT products have the following advantages in terms of process and product.

In terms of process, the invention confers highly efficient crosslinking between HA and BDDE using a lower amount of crosslinking agent (in particular a molar ratio BDDE/ HA of 0.075:1) as compared to Galderma and Allergan brands. The reaction is intensified in a planetary centrifuge under vacuum, as provided herein, which optimizes the reaction time from many hours to less than an hour.

The present invention also provides in one embodiment a pre-filled syringe, which is pre-filled with a crosslinked HA product (or a crosslinked HA hydrogel) or an aqueous composition thereof.

The present invention also provides in one embodiment the use of a crosslinked HA hydrogel product in cosmetic surgery, e.g. dermal filling, body contouring and facial contouring, in medical surgery, e.g. dermal filling, body contouring, prevention of tissue adhesion, formation of channels, incontinence treatment, and orthopedic applications, and for hydrating and/or vitalizing the skin. Accordingly, in one embodiment, the present invention relates to the crosslinked HA hydrogel of the present invention for use in cosmetic surgery, e.g. dermal filling, body contouring and facial contouring. The present invention relates to the crosslinked HA hydrogel of the present invention for use in in medical surgery, e.g. dermal filling, body contouring, prevention of tissue adhesion, formation of channels, incontinence treatment, and orthopedic applications. The present invention further relates to the crosslinked HA hydrogel of the present invention for use in hydrating and/or vitalizing the skin. Accordingly, the present invention also relates to the use of the crosslinked HA hydrogel of the present invention in hydrating and/or vitalizing the skin.

The low amount of BDDE and only one degree of crosslinking allows the fabrication of products within a wide range of HA concentrations (i.e., only one percentage of HA modified by BDDE produce different products), such as varying from 12 to 24 mg/g, and possessing varied viscoelastic properties and applications.

In the PBT technology, the final products, which will be placed in the syringe, are formulated with a mixture of 2 types of particles, characterized by two different average diameters, in a ratio of small particles to large particles of 1:4 (preferably understood to be the number ratio), with small particles being characterized by an average diameter of about 150 µm (wherein the term "about" is generally understood to preferably mean ± 10% of the given numerical value, more preferably ± 5% of the given numerical value, even more preferably ± 2% of the given numerical value, still more preferably ± 1% of the given numerical value, again more preferably the given numerical value) of diameter and large particles being characterized by an average diameter varying from 400 to 800 µm. The particle distribution and composition, in particular the size balance in the formulation, controls the texture, cohesiveness, viscoelasticity, durability, and flexibility of the products, distinguished to meet specific functions of dermal filler application sites.

With a broader and more controlled range of particle sizes, PBT technology may offer a greater range of textures, cohesiveness, viscosity, viscoelasticity, durability, and flexibility to its products.

In addition to textures, cohesiveness, viscosity, durability, and viscoelasticity in PBT, formulations may also be characterized by their flexibility. This property predicts the behavior of hydrogels after application, i.e., the ability to withstand greater or lesser tensions from facial movements without breaking their structure.

Flexibility is defined as the highest tension supported by the hydrogel before the rupture of its structure. In the intact hydrogel, viscoelasticity is greater than viscosity, due to the high elastic portion over fluid portion, provided by the crosslinking). This situation is reversed in rupture stress, with a drop in viscoelasticity and an increase in viscosity.

Another object of the invention is using the hydrogel formulation as a dermal filler for treating wrinkles. Thus, in a further embodiment, the present invention relates to a cosmetic (non-therapeutic) use of the crosslinked HA hydrogel of the present invention, in particular as a dermal filler for treating wrinkles. Further, in one embodiment, the present invention relates to a dermal filler comprising the crosslinked HA hydrogel of the present invention. In accordance with the foregoing, the present invention further relates to the use of the crosslinked HA hydrogel of the present invention in the manufacture of a dermal filler of the present invention.

As it is to be understood herein, treating wrinkles may refer to reducing wrinkles or removing wrinkles, preferably reducing wrinkles (as in, reducing their number, size and/or extent).

The present invention also provides an aqueous composition comprising a crosslinked HA product of the present invention, and optionally a buffering agent and/or a tonicity agent.

The present invention also provides pre-filled syringes, which are pre-filled with a crosslinked HA product or an aqueous composition thereof.

The present invention also provides a crosslinked HA hydrogel product of the present invention for use in cosmetic surgery, e.g., dermal filling, body contouring, and facial contouring, in medical surgery, e.g., dermal filling, body contouring, prevention of tissue adhesion, formation of channels, incontinence treatment, and orthopedic applications, and for hydrating and/or vitalizing the skin.

The term "washing" as referred to herein, is conventional and apparent to the skilled person. In particular, the skilled person is in a position to execute washing, as defined herein, until the excess BDDE is removed from the material.

The invention is illustrated in the following examples. These serve merely illustrative purposes and are not meant to be construed as limiting in any way the scope of the present invention, which is defined by the appended claims.

### Examples

### Example 1

### Synthesis of HA Dermal Filler with Low Concentration of BDDE

NaOH 0.25 mol/L is previously prepared in distilled water, and 9 mL of the alkaline solution is transferred to a planetary centrifuge recipient SMIDA TMV-200T. Then, 1 g of NaHA powder (MW 10⁶ Da) is weighed and transferred to the recipient containing NaOH for dissolution in a planetary centrifuge for 20 min at 2000 rpm and under 10 kPa of vacuum. After complete dissolution, a highly viscous, homogeneous, and transparent solution of NaHA (~12%, m/m) is obtained.

The crosslinking is made by pipetting 34 µL of BDDE into the NaHA alkaline solution (BDDE-HA molar ratio of 0.075:1) and mixing in a planetary centrifuge for 45 min at 2000 rpm and under 10 kPa of vacuum. The resulting mixture is a highly crosslinked gel with a compact and yellowish aspect, with a temperature of 45 ± 5 °C and weight of 3.1 ± 0.7 g. The crosslinked hydrogel is then immersed in 20 mL of phosphate buffer (PBS) at 4 °C to stop the reaction and stabilize the pH.

The partially swollen hydrogel and PBS are transferred to a dialysis membrane with a cut-off of 14 kDa with an available volume of approximately 94 cm³. The membrane is then placed in a tank containing 500 mL of PBS, and the hydrogel gel is dialyzed against PBS for 48 h to remove unreacted BDDE. The washing step is made in a continuous flow system comprising inlet and outlet pumps connected to the tank at 2 mL/min.

After dialysis, the hydrogel is highly cohesive and possesses a neutral pH (7.2-7.4). Then, the hydrogel is weighted and cut into small pieces. HA concentration is corrected with water for injection (WFI) by letting it swell overnight. The final hydrogel mass is corrected according to the desired product, which can vary from 12 to 24 mg/g (Table 1.1), conferring different properties to the products.

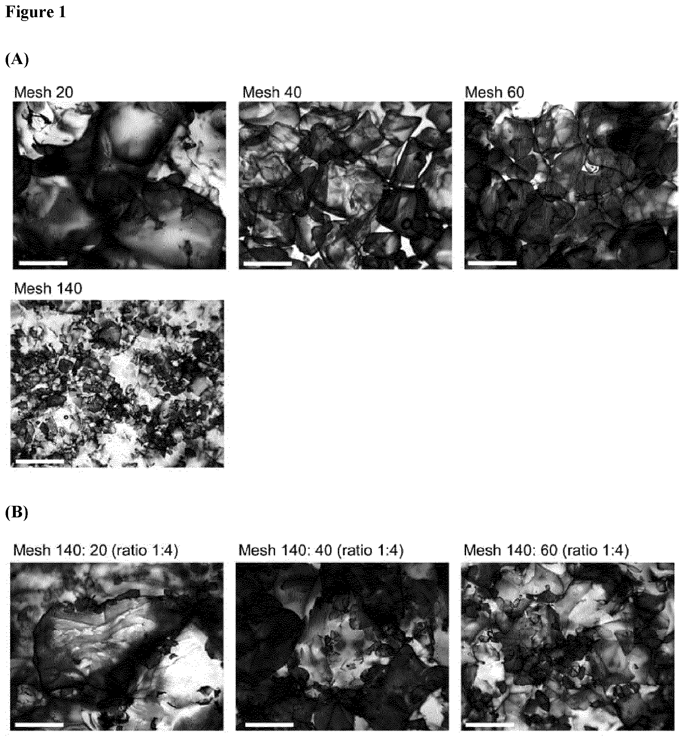

The swelled hydrogel is homogenized into large particles using 60, 40, or 20 mesh sieves to obtain particles with 400, 600, or 800 µm of diameter, respectively (Table 1.2). Then, approximately 24% of the particulate hydrogel is homogenized into small particles using a sieve of mesh 140 to obtain particles with 150 µm of diameter (Figure 1).

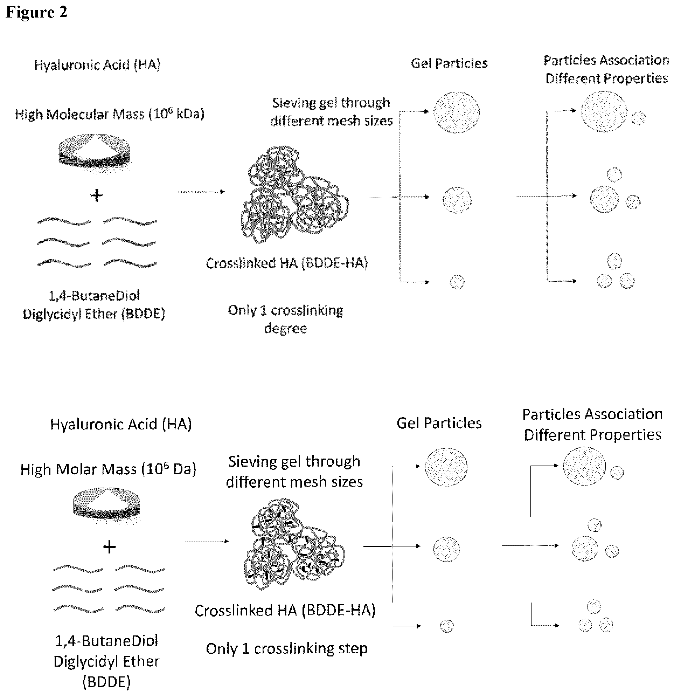

The PBT hydrogel is fabricated by mixing small particles and large particles in the 1:4 (m/m) ratio, with the hydrogel corresponding to 20% of small particles and 80% of large particles (FIG 2). The mixture is made in a planetary centrifuge for 10 min at 1000 rpm.

Non-crosslinked HA is added to the mixture to ensure greater cohesiveness stability, and to facilitate product extrusion. A previously prepared solution of NaHA powder in WFI is then transferred to the PBT hydrogel to obtain a final concentration of free HA (non-crosslinked) of 1% (m/m). The mixture is made in a planetary centrifuge for 10 min at 1000 rpm.

After this step, an anesthetic, such as lidocaine, can be added to the mixture up to the desired concentration, for example, 3 mg/mL (0.3%, m/m), followed by pH correction to 7.2-7.4. The mixture can be made in a planetary centrifuge for 10 min at 1000 rpm.

The resulting PBT hydrogel is filled into sterile syringes of 1 mL and autoclaved for 5 min at 121 °C.

**Table 1.1. Final hydrogel mass according to the HA concentration of the desired product.**

| HA concentration (mg/g) | Final gel mass (g) |
|---|---|
| 12.0 | 83.3 |
| 15.0 | 66.7 |
| 17.5 | 57.1 |
| 20.0 | 50.0 |
| 24.0 | 41.7 |

**Table 1.2. Mean particles size ± standard deviation and polydispersity after sieving swelled hydrogels using different mesh sizes.**

| | Mesh 20 | Mesh 40 | Mesh 60 | Mesh 140 |
|---|---|---|---|---|
| Particles size (µm) | 800.6 ± 61.3 | 603.4 ± 49.8 | 455.7 ± 32.3 | 156.9 ± 20.6 |
| Polydispersity | 0.006 | 0.007 | 0.005 | 0.017 |

### Example 2

### Degree of Modification (MoD) of PBT Dermal Fillers

HA dermal fillers with the particle balance technology (PBT) is based on only 1 crosslinking degree, using a low amount of BDDE (BDDE-HA molar ratio of 0.075: 1) to obtain a wide variety of products. The following sterilized PBT formulations were tested as their crosslinking degree, analyzed by ¹H NMR spectroscopy.

PBT hydrogel was weighted (1 g) and transferred to a 15-mL tube. Hyaluronidase enzyme was pipetted into the gel for a final concentration of 30 U per 0.1 g of hydrogel. The tube was left at 37 °C up to total gel degradation, and the enzyme denatured at 100 °C for 30 min. The hydrogel was filtered in a 0.2 µm filter, frozen at -80 °C, and lyophilized for 24 h. The degraded hydrogel was resuspended in D₂O, and ¹H NMR spectra were recorded on a Bruker 500 MHz instrument at 30 °C.

MoD was calculated by integrating signals at δ 2 ppm from N-acetyl of disaccharide units and at δ 1.6 ppm from BDPE residues. Then, the ratio between integrals was calculated according to the equation: MoD (%) = (I δ1,6 ppm/ 4) / (I õ2ppm/ 3) * 100.

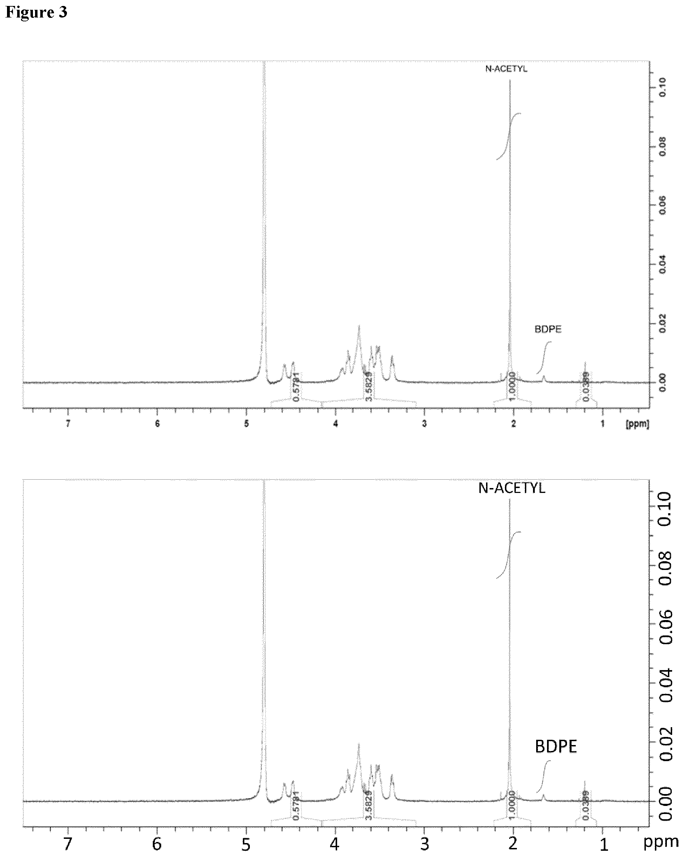

FIG 3 shows the ¹H NMR spectra degraded PBT gel, with an intense peak at 2 ppm and a low-intensity peak at 1.6 ppm. The integration of peaks was made, and MoD was calculated. The MoD was found to be 6.15 ± 0.7%.

### Example 3

### Viscoelastic Properties of PBT Dermal Fillers

Properties of HA products fabricated in accordance with the method described herein are shown in Table 2. Viscoelastic properties were measured using a rheometer Anton Paar MCR92 and parallel plate geometry of 25 mm at 25 °C. Oscillatory measurements were performed in the linear region at a stress of 1.188 Pa in a frequency range of 0.1 to 10 Hz.

PBT hydrogels comprise a wide range of products that can vary from 12 to 24 mg of HA per g of gel and different combinations of particle sizes, all fabricated using only 1 crosslinking degree.

Table 2 presents the range of storage modulus (G'), related to the elastic behavior of hydrogel, and loss modulus (G"), related to the viscous behavior of hydrogel. As samples have gel-like behavior, samples will present a G'> G". The extent of the difference between G' and G" values is represented by tan δ (G"/G'). Values below 1 mean the sample is more elastic than fluid. However, the lower the tan δ, the more stable the sample. That is, as tan δ increases, it decreases the capacity of dermal filler to return to its initial shape during face movements and increases its spread.

With the increase of HA concentration in the PBT gels, G' increases, while tan δ decreases due to the higher elastic characteristic of hydrogels with high HA in the formulation. Hydrogels with low HA concentration present a higher fluid behavior, as seen by the increase of their tan δ. For the same HA concentration, viscoelastic properties of hydrogels were modulated by the particle size ratio, with elasticity increasing with the decrease in diameter of larger particles.

The technology provided herein allows the fabrication of HA dermal fillers with a wide range of properties for different applications.

**Table 2. Viscoelasticity of PBT dermal fillers crosslinked using a low amount of BDDE (BDDE-HA molar ratio of 0.075: 1) for different concentrations of HA and particle size combination.**

| HA concentration (mg/g) | Particle size ratio 1:4 (µm) | G' at 1 Hz (Pa) | G" at 1 Hz (Pa) | tan δ at 1 Hz |
|---|---|---|---|---|
| 12 | 150:400 | 423.4 | 77.9 | 0.18 |
| | 150:600 | 397.9 | 74.9 | 0.19 |
| | 150:800 | 272.62 | 46.5 | 0.17 |
| | | | | |
| 15 | 150:400 | 493.4 | 84.2 | 0.17 |
| | 150:600 | 424.3 | 60.6 | 0.14 |
| | 150:800 | 444.8 | 56.3 | 0.13 |
| | | | | |
| 17.5 | 150:400 | 603.2 | 90.1 | 0.15 |
| | 150:600 | 451.2 | 70.7 | 0.16 |
| | 150:800 | 541.1 | 70.1 | 0.13 |
| | | | | |
| 20 | 150:400 | 683.2 | 94.9 | 0.14 |
| | 150:600 | 634.5 | 88.4 | 0.14 |
| | 150:800 | 544.1 | 71.1 | 0.13 |
| | | | | |
| 24 | 150:400 | 727.4 | 110.6 | 0.15 |
| | 150:600 | 892.5 | 122.8 | 0.14 |
| | 150:800 | 849.5 | 88.4 | 0.10 |

### Example 4

### Extrusion Force of PBT Hydrogels

The injectability of PBT gels was tested using their extrusion force through needles of different inner diameters. Tests were performed in homemade equipment, and force was expressed in Newtons (N), directly affected by the degree of crosslinking. All PBT hydrogels were fabricated with only 1 degree of crosslinking, and yet, the force of injection varied with other gel characteristics and properties, such as HA concentration and particle balance.

PBT gels were filled into the 1-mL syringes, autoclaved, and connected to sterilized needles of 27-32G. Syringes were then connected to the homemade equipment and compressed at 12.5 mm/min until hydrogel extrusion. Force values are shown in Table 3 and represent the highest peak measured during extrusion.

PBT hydrogels with HA concentration of 12 mg/g present a higher viscous portion and, therefore, were extruded through a 32G needle, while PBT hydrogels with 15 and 17.5 mg/g of HA were extruded through a 30G needle. PBT gels with the highest concentration of HA, 20 and 24 mg/g, possess lower viscous portions and are more rigid; therefore, they were extruded through a 27G needle.

Particle size directly influenced the hydrogel extrusion, and the force required to extrude gels composed of particles balanced 150/ 800 µm was much higher than formulations composed of smaller particles, especially through 32G and 30G needles. Therefore, using a 27G needle would be recommended for this condition. PBT gels with higher HA concentrations, 20 and 24 mg/g, were easily extruded through 27G needles and presented extrusion force between 10 and 30 N, depending on the particle size combination.

**Table 3. Extrusion force of PBT gels of different HA concentration and particles size balance.**

| HA concentration (mg/g) | Particle size ratio 1:4 (µm) | Needle (G) | Extrusion force (N) |
|---|---|---|---|
| 12 | 150:400 | 32 | 36.5 |
| | 150:600 | | 35.6 |
| | 150:800 | | 78 |
| | | | |
| 15 | 150:400 | 30 | 25.6 |
| | 150:600 | | 18.5 |
| | 150:800 | | 57 |
| | | | |
| | 150:400 | 30 | 22.3 |
| | 150:600 | | 33.1 |
| | 150:800 | | 48.6 |
| | | | |
| 20 | 150:400 | 27 | 18.9 |
| | 150:600 | | 13.0 |
| | 150:800 | | 23.9 |
| | | | |
| 24 | 150:400 | 27 | 12.5 |
| | 150:600 | | 16.3 |
| | 150:800 | | 29.3 |

### Example 5

### Synthesis of HA Hydrogels with Higher Concentrations of BDDE and Particle Sizes from an Ultra-Turrax Equipment

This example shows the PBT technology applied to HA hydrogels with BDDE-HA molar ratios 0.1:1 to 0.5:1. The hydrogels were obtained with high MW HA, average 10⁶ Da and one crosslink degree (same BDDE-HA ratio); Adjustment of the total HA concentration with crosslinked and free HA (i.e., non-crosslinked HA).

Table 4.1 shows the size combination (600 µm and 150 µm) in various proportions, resulting in an extensive range of viscoelasticity represented by G', the storage modulus, characterizing the viscoelasticity of the hydrogels. Other size combinations and proportions could be obtained, amplifying the range of properties. Therefore, according to the invention, the technology offers opportunities for fabricating dermal fillers with different properties for an extensive range of applications.

**Table 4.1**

| BDDE-HA molar ratio | HA concentration (mg/mL) | Free HA (%) | Particle size ratio (600-150 µm) | Needle (Gauge) | G' 0.1-10 Hz (Pa) |
|---|---|---|---|---|---|
| 0.1:1 | 12 | 17 | 0:1 | 32 | 4-85 |
| 0.1:1 | 12 | 17 | 1:1 | 30 | 190-320 |
| 0.1:1 | 12 | 17 | 4:1 | 27 | 500-680 |
| 0.1:1 | 15 | 33 | 1:1 | 30 | 140-275 |
| 0.1:1 | 15 | 33 | 4:1 | 27 | 300-480 |
| 0.5:1 | 17.5 | 14 | 1:1 | 30 | 580-1170 |
| 0.5:1 | 17.5 | 14 | 4:1 | 27 | 785-1320 |
| | | | | | |
| 0.5:1 | 20 | 33 | 1:1 | 27 | 550-1140 |
| 0.5:1 | 20 | 33 | 4:1 | 27 | 820-1200 |
| 0.5:1 | 24 | 37.5 | 1:0 | 27 | 840-1370 |

Table 4.2 shows the parameters of Ultra Turrax, such as speed and time, calibrated for fabricating hydrogels with particles of specific diameter for BDDE-HA molar ratio of 0.1:1 and 0.5:1.

**Table 4.2**

| Speed (rpm) | Time (min) | Particle size (µm) |
|---|---|---|
| 3000 | 3 | 600 ± 120 |
| 4000 | 5 | 500 ± 150 |
| 4000 | 10 | 400 ± 110 |
| 8000 | 20 | 250 ± 70 |
| 10000 | 20 | 150 ± 45 |

### References

Rzany B, et al. "European Consensus on the Use of Injectable Poly-L-Lactic Acid for Aesthetic Procedures." Dermatologic Surgery. 2018;44(Suppl 1):S38-S51.
Narins RS, et al. "Consensus Recommendations for Combined Aesthetic Interventions Using Botulinum Toxin, Fillers, and Microfocused Ultrasound in the Neck, Décolletage, Hands, and Other Areas." Dermatologic Surgery. 2016;42(10):1199-1208.
Burgess CM. "Overview of Hyaluronic Acid Fillers: History, Safety, and Recent Advances." Journal of Drugs in Dermatology. 2018;17(2):s24-s28.
Micheels P, et al. "Structural Characteristics and Rheological Properties of HA Fillers: A Review." Journal of Cosmetic Dermatology. 2019;18(2):486-494.
US 11058640 B1, Hyaluronate compositions and soft tissue fillers.
US 20160376382 A1, Process for preparing a cross-linked hyaluronic acid product.
US 8394782 B2 Polysaccharide Gel Formulation having increased longevity.
US 7,741,476 B2 Cross-linking of low and high molecular weight polysaccharides preparation of injectable monophasic hydrogels and polysaccharides and hydrogels thus obtained.
US 6921819 B2, Polysaccharide crosslinking hydrogel preparation polysaccharide(s) and hydrogel(s) uses thereof.
Kenne L et al "Modification and cross-linking parameters in hyaluronic acid hydrogels-Definitions and analytical methods" Carbohydrate Polymers. 2012, Vol 91, Num 1, pp 410-418, 9 p ; ref: 1/4 p
Faivre J et al "Crosslinking hyaluronic acid soft-tissue fillers: current status and perspectives from an industrial point of view" Expert Rev Med Devices 2021 Dec;18(12):1175-1187. doi: 10.1080/17434440.2021.2014320. Epub 2022 Jan 10.
Xue Y et al "Synthesis of hyaluronic acid hydrogels by crosslinking the mixture of high-molecular-weight hyaluronic acid and low-molecular-weight hyaluronic acid with 1,4-butanediol diglycidyl ether" RCS Advance 2020 10(12) : 7206-7213
Micheels P. et al "Effect of different crosslinking technologies on hyaluronic acid behavior a visual and microscopic of seven hyaluronic acid gel" J Drugs Dermatol 2016 May 1;15(5):600-6.
Edsman K "Gel Properties of Hyaluronic Acid Dermal Fillers" Dermatologic Surgery 38(7 pt2):p 1170-1179, July 2012. DOI: 10.1111/j . 1524-4725.2012.02472.x
Kablik J." Comparative physical properties of hyaluronic acid dermal fillers" Dermatol Surg. 2009 Feb;35 Suppl 1:302-12.doi: 10.1111/j.1524-4725.2008.01046.x.
Li D. "A multi-center comparative efficacy and safety study of two different hyaluronic acid fillers for treatment of nasolabial folds in a Chinese population" Journal of Cosmetic Dermatology, Volume18, Issue3 June 2019 Pages 755-761.
Allemann I.B. "Hyaluronic acid gel (Juvéderm) preparations in the treatment of facial wrinkles and folds" Clin Interv Aging. 2008;3(4):629-34 doi: 10.2147/cia.s3118.
Duranti F. "Injectable hyaluronic acid gel for soft tissue augmentation. A clinical and histological study" Dermatol Surg. 1998 Dec;24(12):1317-25 doi: 10.1111/j.1524-4725.1998.tb00007.x.
Segurado M.A. "An expert consensus report on the clinical use of the Vycross® hyaluronic acid VYC-25 L filler" J Cosmet Dermatol. 2021 Oct ;20(10):3155-3164. doi: 10.1111/jocd. 14398.
Brandt F.S. "Hyaluronic acid gel fillers in the management of facial aging" Clin Interv Aging. 2008;3(1):153-9. doi: 10.2147/cia.s2135.
Fallacara A. "Hyaluronic Acid Fillers in Soft Tissue Regeneration" Facial Plast Surg. 2017 Feb;33(1):87-96 doi: 10.1055/s-0036-1597685.
Choi M.S. "Basic rheology of dermal filler" Arch Plast Surg. 2020 Ju1;47(4):301-304 doi: 10.5999/aps.2020.00731.
Fundarò S.P. "The Rheology and Physicochemical Characteristics of Hyaluronic Acid Fillers: Their Clinical Implications" Int J Mol Sci 2022 Sep 10;23(18):10518. doi: 10.3390/ijms231810518.

## Claims

1. A process of manufacturing crosslinked hyaluronic acid (HA) hydrogels comprising a mixture of two particle sizes of 150 µm and from 400 µm to 800 µm and having HA concentration of 12 to 24 mg/g, the process comprising the following steps:
a/ crosslinking of HA having an average MW of 10⁶ Da with molar ratio of crosslinking agent 1,4-butanediol diglycidyl ether (BDDE) to HA of 0.075: 1;
b/ conducting reaction of HA and BDDE in a planetary centrifuge under vacuum for less than 1 hour;
c/ washing the crosslinked HA and swelling to achieve the HA concentration of from 12 to 24 mg of HA per g of gel;
d/ homogenizing the crosslinked and swelled HA into particles using sieves of different mesh sizes and obtaining gel particles of two specific diameters between 150 and 800 µm, as measured by optical microscopy;
e/ isolating a batch of HA particles with an average diameter of 150 µm and a batch of HA particles with an average diameter ranging from 400 to 800 µm;
f/ combining particles **characterized by** a diameter of 150 µm with particles of a diameter of from 400 to 800 µm to fabricate the hydrogels; and
g/ mixing particles of different sizes in a planetary centrifuge.

2. A process of manufacturing crosslinked hyaluronic acid (HA) hydrogels, comprising the steps of:
h/ crosslinking of HA having a MW of 10⁶ Da with the crosslinking agent 1,4-butanediol diglycidyl ether (BDDE) in the range of 0.1:1 to 0.5:1 BDDE-HA molar ratios containing only 1 crosslinking degree;
i/ conducting the reaction of HA and BDDE in a reactor with mechanical stirring at controlled temperature and a processing time higher than 1 hour; and
j/ obtaining particles using a high shear homogenizer, for example an ultra-turrax equipment, with pre-calibrated rotation speed and application time.

3. The process according to claim 1, wherein particles of different average diameters are mixed and combined in step f/ with a proportion of 1:4 of small particles **characterized by** the average diameter of 150 µm to large particles **characterized by** the average diameter ranging from 400 to 800 µm, and then submitted to intensified mixing in step g/ to homogenization of particles.

4. The process according to claims 1 or 3, wherein the particles combined in step f/ are supplemented with about 1% w/w of free non-crosslinked HA.

5. The process according to any one of claims 1 or 3 to 4, wherein the HA used is in the form of sodium salt of HA (NaHA) with an average MW of 10⁶ Da.

6. The process according to any one of the preceding claims, wherein the crosslinking and particles mixing are performed in a planetary centrifuge.

7. The process according to any one of the preceding claims, wherein an anesthetic such as lidocaine is then incorporated in the hydrogel.

8. The process according to any one of the preceding claims, wherein the obtained hydrogel is sterilized, for example sterilized in an autoclave.

9. A cross-linked HA hydrogel obtainable according to the process of any one of claims 1 to 8.

10. An aqueous composition comprising the cross-linked HA hydrogel of claim 9, and optionally a buffering agent and/or a tonicity agent.

11. A pre-filled syringe, pre-filled with the cross-linked HA product of claim 9 or the aqueous composition thereof as defined in claim 10.

12. The crosslinked HA hydrogel of claim 9 for use in cosmetic surgery, e.g. in dermal filling, body contouring and facial contouring, in medical surgery, e.g. dermal filling, body contouring, prevention of tissue adhesion, formation of channels, incontinence treatment, and orthopedic applications, or for hydrating and/or vitalizing the skin.

13. Cosmetic use of the crosslinked HA hydrogel of claim 9 or the composition of claim 10 as dermal filler for the treatment of wrinkles.

14. A dermal filler containing a crosslinked HA hydrogel of claim 9.
